# EUROPÄISCHE PATENTSCHRIFT

(11) **EP 3 393 591 B1**
(45) Veröffentlichungstag und Bekanntmachung des Hinweises auf die Patenterteilung: **08.04.2020**
(21) Anmeldenummer: 16808962.1
(22) Anmeldetag: 01.12.2016
(51) Int. Cl.: A61Q 5/04, A61K 8/26, A61K 8/19, A61K 8/73

(54) **GERUCHREDUZIERTES VERFAHREN ZUR PERMANENTEN UMFORMUNG VON KERATINFASERN**
ODOR-REDUCED PROCESS FOR THE PERMANENT FORMING OF KERATINE FIBERS
PROCEDE DE TRANSFORMATION PERMANENTE DES FIBRES KERATINIQUES, ODEUR RÉDUITE

(30) Priorität: 21.12.2015 DE 102015226174
(43) Veröffentlichungstag der Anmeldung: 31.10.2018
(73) Patentinhaber: Henkel AG & Co. KGaA, 40589 Düsseldorf (DE)
(72) Erfinder: MANNECK, Hartmut, 23858 Barnitz (DE); HOEPFNER, Stefan, 22523 Hamburg (DE); SCHWEINSBERG, Matthias, 40764 Langenfeld (DE)
(86) Internationale Anmeldenummer: PCT/EP2016/079379
(87) Internationale Veröffentlichungsnummer: WO 2017/108362

(56) Entgegenhaltungen:
- EP-A1- 0 114 414
- EP-A2- 0 085 894
- EP-A2- 0 256 462
- DE-A1-102006 026 305

## Beschreibung

### Technischer Hintergrund

Gegenstand der vorliegenden Erfindung ist ein Verfahren zur permanenten Umformung, insbesondere zum Glätten oder zum Dauerwellen, von keratinischen Fasern, insbesondere menschlichen Haaren, durch das die bei Dauerwell- und Haarglättungsmitteln auftretenden intensiven und reizenden Gerüche der schwefelhaltigen Reduktionsmittel (Thioglycolat, Cystein etc.) und des Ammoniaks minimieren.

Die dauerhafte Verformung von Keratinfasern wird üblicherweise so durchgeführt, dass man die Faser mit Hilfe von mechanischen Verformungshilfsmitteln (Wickler, Papilloten) verformt und die Verformung durch geeignete Hilfsmittel festlegt. Vor und/oder nach dieser Verformung behandelt man die Faser mit der wässrigen Zubereitung einer Keratin reduzierenden Substanz und spült nach einer Einwirkungszeit mit Wasser oder einer wässrigen Lösung. In einem zweiten Schritt behandelt man dann die Faser mit der wässrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wässrige Zubereitung der Keratin reduzierenden Substanz ist üblicherweise alkalisch eingestellt, damit zum einen ein genügender Anteil der Thiolfunktionen deprotoniert vorliegt und zum anderen die Faser quillt und auf diese Weise ein tiefes Eindringen der Keratin reduzierenden Substanz in die Faser ermöglicht wird. Die keratinreduzierende Substanz spaltet einen Teil der Disulfid-Bindungen des Keratins zu -SH-Gruppen, so dass es zu einer Lockerung der Peptidvernetzung und infolge der Spannung der Faser durch die mechanische Verformung zu einer Neuorientierung des Keratingefüges kommt. Unter dem Einfluss des Oxidationsmittels werden erneut Disulfid-Bindungen geknüpft, und auf diese Weise wird das Keratingefüge in der vorgegebenen Verformung neu fixiert. Ein bekanntes derartiges Verfahren stellt die Dauerwell-Behandlung menschlicher Haare dar. Dieses kann sowohl zur Erzeugung von Locken und Wellen in glattem Haar als auch zur Glättung von gekräuselten oder ethnischen Haaren angewendet werden.

Eine Verbindung gilt im Sinne der Erfindung als Keratin reduzierend, wenn eine Lösung von 50 mmol dieser Verbindung in 100 g Wasser bei 20°C und einem pH-Wert von pH 8 (eingestellt mit 2-Aminoethanol) Disulfid-Bindungen des Keratins zu -SH-Gruppen spaltet.

Übliche Keratin reduzierende Substanzen sind ausgewählt aus Thioglykolsäure und deren Salzen, und /oder Thiomilchsäure und deren Salzen und/oder Cystein und/oder Acetylcystein und/oder Cysteamin und deren Salzen sowie Mischungen derselben. Diese Substanzen weisen einen deutlichen und sehr unangenehmen schwefeligen Geruch auf. Siehe als Stand der Technik z.B. EP 0 114 414 A1, EP 0 085 894, EP 0 256 462.

Um eine zufriedenstellende Keratin reduzierende Leistung zu entfalten, benötigen die erfindungsgemäßen Mittel bei der Anwendung in der Regel einen alkalischen oder nur schwach sauren pH-Wert. Insbesondere bei pH-Werten im Bereich von 7 bis 9,5 werden optimale Ergebnisse erzielt. Zur Einstellung dieser pH-Werte ist bis zum heutigen Zeitpunkt Ammoniak das Alkalisierungsmittel der Wahl. Mit Ammoniak lässt sich nicht nur der für die Farbstoffbildung notwendige pH-Bereich einstellen, sondern Ammoniak sorgt auch in stärkerem Maß als alle anderen bekannten Alkalisierungsmittel für eine Quellung der Haare. Gleichzeitig wirkt Ammoniak - ebenfalls in stärkerem Ausmaß als alle anderen handelsüblichen Alkalisierungsmittel - als Penetrations- bzw. Penetrationshilfsmittel. Die mit dem Einsatz von Ammoniak verbundenen anwendungstechnischen Vorteile sind so vielfältig, dass Ammoniak trotz seines unangenehmen, stechenden Geruchs in einer Vielzahl handelsüblicher Glätt- und Wellmittel verwendet wird.
Eine prinzipielle Möglichkeit zur Reduktion des schwefeligen Geruchs oder des Ammoniakgeruchs besteht im Zusatz von speziellen Parfümstoffen, welche die Gerüche überdecken sollen. Parfümstoffe können jedoch unter den alkalischen Lagerbedingungen instabil sein, so dass die Gefahr besteht, dass die Duftstoffe während der Lagerung abgebaut oder strukturell verändert werden, was sich auch in einer unvorhersehbaren Änderung des Geruchs niederschlägt. Da entsprechende Veränderungen oftmals erst nach mehreren Monaten oder sogar Jahren wahrnehmbar werden, wird der Einsatz von neuen bzw. unbekannten Parfums als problematisch eingestuft.
Eine weitere prinzipielle Möglichkeit zur Reduktion des schwefeligen Geruchs oder des Ammoniakgeruchs besteht in einer Optimierung der Formulierung. Hierbei gilt es, die Träger-Bestandteile der Formulierung so auszuwählen, dass diese einen optimalen Rückhalt der Keratin reduzierenden Substanzen in der Formulierung gewährleisten und auf diese Weise deren Geruch minimieren. Es ist jedoch ebenfalls bekannt, dass die Formulierung, die ggf. in ihr enthaltenen Fettstoffe, ihre Emulgatoren, Tenside und ihre Viskosität wesentlichen Einfluss auf die keratolytische Leistung nehmen. Bei der Modifikation der Formulierung muss eine Verschlechterung der keratolytischen Leistung daher auf jeden Fall vermieden werden.
Insbesondere die dauerhafte Geruchsminimierung über die gesamte Anwendungsdauer ist nur sehr schwierig zu erreichen. Die Zeitspanne, innerhalb der sich der Anwender in Kontakt mit dem Well- oder Glättmittel befindet, reicht von der Auftragung des Mittels auf die Haare und den Zeitraum des Einwirkens bis zum Auswaschen der Formulierung. Bei üblichen Einwirkzeiten von 5-60 Minuten, bevorzugt 10-30 Minuten kann der gesamte Prozess bis zu 75 Minuten Zeit in Anspruch nehmen. Eine geruchliche Abdeckung der Keratin reduzierenden Substanzen oder Ammoniaks, die über diesen gesamten Zeitraum wirksam ist, stellt die höchste Herausforderung dar. Gerade auf diesem Feld besteht noch starker Optimierungsbedarf, und eine optimale Möglichkeit zur dauerhaften Reduktion des Geruchs der Keratin reduzierenden Substanzen ist aus dem Stand der Technik bislang nicht bekannt.

Aufgabe der vorliegenden Erfindung war daher die Bereitstellung eines Verfahrens zur permanenten Umformung, insbesondere zum Glätten oder zum Dauerwellen, von keratinischen Fasern, insbesondere menschlichen Haaren, mit reduziertem schwefeligen Geruch oder mit reduziertem Ammoniakgeruch.

Hierbei sollen die erfindungsgemäß verwendeten chemischen Umformungsmittel keine Einbußen in ihrer Keratin reduzierenden Leistung aufweisen.

Es war hierbei insbesondere die Aufgabe der vorliegenden Erfindung, über die gesamte Anwendungsdauer eine Reduktion des schwefeligen Geruchs und/oder des Ammoniakgeruchs zu erzielen. Auch nach maximal zweistündigem Kontakt sollte die geruchliche Wahrnehmung der Keratin reduzierenden Substanzen und/oder des Ammoniaks immer noch wirksam minimiert sein.

Gegenstand der vorliegenden Anmeldung ist ein Verfahren zur permanenten Umformung, insbesondere zum Glätten oder zum Dauerwellen, von keratinischen Fasern, insbesondere menschlichen Haaren, durch das die bei Dauerwell- und Haarglättungsmitteln auftretenden intensiven und reizenden Gerüche der schwefelhaltigen Reduktionsmittel (Thioglycolat, Cystein etc.) oder des Ammoniaks minimiert werden, ohne die Umformleistung der erfindungsgemäß verwendeten Keratinreduktionsmittel zu beeinträchtigen. Die Erfindung ist ausschliesslich wie in den Ansprüchen definiert. Teile der Beschreibung die nicht unter den Umfang der Ansprüche fallen, aber irrtümlich als Teil der Erfindung gekennzeichnet sind, sind lediglich als Teil der Offenbarung zu betrachten.

Hierzu kommen in dem erfindungsgemäßen Verfahren zum einen mindestens ein ausgewähltes Salz eines mehrwertigen Metallions, ausgewählt aus Calcium-, Strontium-, Barium- und Aluminiumsalzen und unter bestimmten Voraussetzungen ausgewählt aus Kaliumsalzen, jeweils mit einer Wasserlöslichkeit bei 20°C von mindestens 500 mg/l, zum anderen mindestens ein ausgewähltes Polysaccharid zum Einsatz, das mit den mehrwertigen Metallionen auf den Keratinfasern einen gelierten Film bildet. Ohne an diese Theorie gebunden sein zu wollen, wird vermutet, dass dieser Film als Barriere wirkt, die die Freisetzung des Ammoniaks und der schwefeligen Verbindungen in die Umgebung verlangsamt und somit deren Gerüche reduziert.

Unter den Begriffen "keratinische Fasern" und "Keratinfasern" sind erfindungsgemäß bevorzugt menschliche Haare, daneben aber auch Pelze, Wolle und Federn zu verstehen.

Unter "anschließend" wird erfindungsgemäß bevorzugt ein Zeitraum von 1 bis 600 Sekunden verstanden.

Gegenstand der vorliegenden Erfindung ist ein Verfahren zur permanenten Umformung, insbesondere zum Glätten oder zum Dauerwellen, von keratinischen Fasern, das durch folgende Verfahrensschritte gekennzeichnet ist:
(i) Verformen der Fasern unter Zuhilfenahme von mechanischen Verformungshilfsmitteln, wie Wickler oder Papilloten, nach, vor oder während des Schritts (ii),
(ii) Auftragen einer chemischen Umformzusammensetzung (KR) Auftragen einer chemischen Umformzusammensetzung (KR) zum Glätten oder zum Dauerwellen auf die Fasern, enthaltend
   - mindestens eine Keratin reduzierende Verbindung, ausgewählt aus Thioglykolsäure und deren Salzen und / oder Thiomilchsäure und deren Salzen und/oder Cystein und/oder Acetylcystein und/oder Cysteamin und deren Salzen sowie Mischungen dieser Keratin reduzierenden Verbindungen, weiterhin
   - Wasser, weiterhin
   - mindestens ein Alkalisierungsmittel,
   anschließend
(iii) Auftragen einer Polysaccharid-Zusammensetzung (D), enthaltend
   iii.1 mindestens ein Polysaccharid, das mit Calciumionen in wässrigem Medium ein Gel bilden kann und ausgewählt ist aus kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
   iii.2 50 - 99,9, bevorzugt 60 bis 95, besonders bevorzugt 80 bis 90 Gew.-% Wasser, jeweils bezogen auf das Gewicht der Zusammensetzung (D),
   iii.3 optional mindestens ein Alkalisierungsmittel, das bevorzugt ausgewählt ist aus pulverförmigen Natriumsilikaten, insbesondere pulverförmigen Natriummetasilikaten,
   iii.4 optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),
   iii.5. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),
   wobei optional die Polysaccharid-Zusammensetzung (D) durch Vermischen einer festen, bevorzugt pulverförmigen, Polysaccharid-Zusammensetzung (D'), enthaltend iii.1 und optional iii.3 und/oder optional iii.4, mit der Komponente iii.2 und optional mit der Komponente iii.5 *in situ* oder 0,01 bis 24 Stunden vor Durchführung des erfindungsgemäßen Glättungs- oder Umformverfahrens hergestellt wird,
(iv) Spülen der Fasern nach einer Einwirkzeit Z1, bevorzugt nach 5-60 Minuten, besonders bevorzugt nach 10-30 Minuten und gegebenenfalls Trocknen,
(v) anschließend Auftragen eines Fixiermittels, enthaltend mindestens ein Oxidationsmittel, auf die Fasern und Abspülen nach einer Einwirkzeit Z2, bevorzugt nach 1-30 Minuten, besonders bevorzugt nach 5-20 Minuten,
dadurch gekennzeichnet, dass in einer ersten Variante das Verfahren die Applikation von mindestens einem Salz eines mehrwertigen Metallions, ausgewählt aus Calcium-, Strontium-, Barium- und Aluminiumsalzen mit einer Wasserlöslichkeit bei 20°C von mindestens 500 mg/l, umfasst, oder in einer zweiten Variante für den Fall, dass das vorgenannte Polysaccharid in der Polysaccharid-Zusammensetzung (D), das mit Calciumionen ein Gel bilden kann, kappa-Carragheenan oder eines seiner Salze umfasst, das Verfahren die Applikation von mindestens einem Salz, ausgewählt aus Kalium-, Calcium-, Strontium-, Barium- und Aluminiumsalzen mit einer Wasserlöslichkeit bei 20°C von mindestens 500 mg/l enthält; umfasst, wobei in beiden Varianten diese Calcium-, Strontium-, Barium- und Aluminiumsalze (1. Variante) bzw. Kalium-, Calcium-, Strontium-, Barium- und Aluminiumsalze (2. Variante) nicht in der Polysaccharid-Zusammensetzung (D) enthalten sind.

Das Fixiermittel enthält mindestens ein Oxidationsmittel, das bevorzugt ausgewählt ist aus Wasserstoffperoxid in wässriger Lösung oder Emulsion, wobei diese Zusammensetzung zur Stabilisierung des Wasserstoffperoxids einen sauren pH-Wert im Bereich von 2,5 bis 6,5, bevorzugt im Bereich von 3 - 5,5, besonders bevorzugt im Bereich von 3,5 bis 5,0, aufweist, jeweils bei 20°C gemessen.

Für das Umformungsergebnis ist es wichtig, dass die Umformzusammensetzung (KR) einen pH-Wert im Bereich von 6,5 bis 10,0, bevorzugt von 7,5 bis 9,5, besonders bevorzugt 8,0 bis 9,0, aufweist, jeweils gemessen bei 20°C. Bei diesen pH-Werten öffnet sich die äußere Keratinfaserschicht optimal zur Aufnahme des Keratinreduktionsmittels und entfaltet sich dessen reduktive Wirkung optimal.

Zur Einstellung der vorgenannten pH-Werte enthält die Umformzusammensetzung (KR) ein oder mehrere Alkalisierungsmittel in einer geeigneten Menge.

### Gel bildendes Salz

Ein wesentlicher Bestandteil erfindungsgemäßer und erfindungsgemäß bevorzugter Umformverfahren ist mindestens ein Salz eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l.
Dieses Salz kann in das erfindungsgemäße Verfahren eingebracht werden, indem es
A. der Umformzusammensetzung (KR) bereits bei der Herstellung zugesetzt wird, oder
B. pulverförmig bereitgestellt wird und der Umformzusammensetzung (KR) *in situ* zugesetzt wird, oder
C. in Form einer Wasser-basierten Gelierzusammensetzung (C) bereitgestellt und der Umformzusammensetzung (KR) *in situ* zugesetzt wird, oder
D. in Form einer Wasser-basierten Gelierzusammensetzung (C) bereitgestellt und nach dem Auftragen der Umformzusammensetzung (KR) auf die Keratinfasern ebenfalls auf die Keratinfasern aufgetragen, insbesondere aufgesprüht, wird.

Falls das gelbildende Salz über mindestens einen der vorstehend genannten Alternativen A, B oder C in das erfindungsgemäße Verfahren eingebracht wird, so wird es nach seiner Applikation auf die Keratinfasern in Kontakt mit mindestens einem ausgewählten Polysaccharid gebracht, das mit Calciumionen ein Gel bilden kann. Dadurch bildet sich ein filmförmiges Gel auf den mit der Umformzusammensetzung (KR) beaufschlagten Keratinfasern, das als Barriere gegen die Freisetzung von Ammoniak- und schwefeligen Gerüchen wirkt und diese Gerüche reduziert.

Falls das gelbildende Salz über die vorstehend genannte Alternative D in das erfindungsgemäße Verfahren eingebracht wird, so kann es vor oder nach seiner Applikation auf die Keratinfasern in Kontakt mit mindestens einem ausgewählten Polysaccharid gebracht werden, das mit Calciumionen ein Gel bilden kann. So kann zuerst die Gelierzusammensetzung (C) und anschließend die Polysaccharid-Zusammensetzung (D) auf die Keratinfasern aufgebracht, insbesondere aufgesprüht werden. Ebenso gut ist es möglich, zuerst die Polysaccharid-Zusammensetzung (D) und anschließend die Gelierzusammensetzung (C) auf die Keratinfasern aufzutragen, insbesondere aufzusprühen. Dadurch bildet sich ein filmförmiges Gel auf den mit der Umformzusammensetzung (KR) beaufschlagten Keratinfasern, das als Barriere gegen die Freisetzung von Ammoniak- und schwefeligen Gerüchen wirkt und diese Gerüche reduziert.

Bevorzugt ist dieses mit Calciumionen gelierende Polysaccharid ausgewählt aus, kappa-Carragheenan, iota-Carragheenan und Pektin und/oder mindestens einem Salz der vorgenannten Polysaccharide, das wiederum ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist.
Das mindestens eine gelbildende Salz weist bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, auf. Das erfindungsgemäß außerordentlich bevorzugte Calciumchlorid hat eine Wasserlöslichkeit von 740 g/l bei 20°C. Andere erfindungsgemäß besonders bevorzugte gelbildende Salze sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein.

### Polysaccharid, ausgewählt aus kappa-Carragheenan, iota-Carragheenan und Pektin, und/oder mindestens ein Salz dieser Polysaccharide

Ein weiterer wesentlicher Bestandteil erfindungsgemäßer und erfindungsgemäß bevorzugter Umformverfahren ist mindestens ein Polysaccharid, das mit Calciumionen in wässrigem Medium ein Gel bildet. Erfindungsgemäß bevorzugte Polysaccharide, die mit Calciumionen in wässrigem Medium ein Gel bilden können, sind ausgewählt aus kappa-Carragheenan, iota-Carragheenan und Pektin sowie mindestens einem Salz der vorgenannten Polysaccharide, das ausgewählt aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist. Wässrige Lösungen dieser Polysaccharide und Polysaccharidsalze zeigen je nach Konzentration eine nur leicht erhöhte Viskosität, bilden aber noch keine festen Gele.

Besonders bevorzugt sind kappa-Carragheenan, das Lithiumsalz von kappa-Carragheenan, das Natriumsalz von kappa-Carragheenan, iota-Carragheenan, das Lithiumsalz von iota-Carragheenan, das Natriumsalz von iota-Carragheenan, das Kaliumsalz von iota-Carragheenan, das Ammoniumsalz von iota-Carragheenan, Pektin, Natriumpektinat, Ammoniumpektinat, Kaliumpektinat und Magnesiumpektinat, sowie Mischungen dieser Substanzen.

Die vorstehend genannten Polysaccharide und/oder die genannten Salze zeigen ein weitgehend pHunabhängiges Verdickungsverhalten. Allerdings gelieren sie im Kontakt mit bestimmten mehrwertigen Ionen, wie Calcium-, Strontium-, Barium- und Aluminiumionen. Darüber hinaus geliert kappa-Carragheenan auch im Kontakt mit Kaliumionen.

Dieses Verhalten wird im Sinne der vorliegenden Anmeldung dazu genutzt, auf den Keratinfasern nach der Applikation der Umformzusammensetzung (KR) einen Film zu erzeugen, der die Freisetzung von Ammoniak- und schwefeligen Gerüchen reduziert.

Für die vier vorgenannten bevorzugten Ausführungsformen des erfindungsgemäßen Umformverfahrens (Alternativen A bis D) ist es besonders bevorzugt, dass das Gewichtsverhältnis von Polysaccharid-Zusammensetzung (D) zur Umformzusammensetzung (KR) im Bereich von 1 : 20 bis 1:2, bevorzugt im Bereich von 1:10 bis 1:5 liegt.

Für die vorgenannten bevorzugten erfindungsgemäßen Umformverfahren gemäß einer der Alternativen C oder D ist es weiterhin besonders bevorzugt, dass das Gewichtsverhältnis von Polysaccharid-Zusammensetzung (D) zur Gelierzusammensetzung (C) im Bereich von 0,2 : 1 bis 2:1, bevorzugt im Bereich von 0,5 : 1 bis 1,5 : 1 liegt und besonders bevorzugt 1:1 beträgt.

Für die vorgenannten bevorzugten erfindungsgemäßen Umformverfahren gemäß einer der Alternativen C oder D ist es weiterhin besonders bevorzugt, dass das Gewichtsverhältnis von Gelierzusammensetzung (C) zur Umformzusammensetzung (KR) im Bereich von 1 : 20 bis 1:2, bevorzugt im Bereich von 1:10 bis 1:5 liegt.

Wenn nach Ablauf der Einwirkzeit Z1 die zur Umformung nötige Keratinreduktion abgeschlossen ist, kann das gesamte Haarbehandlungsmittel in einem einzigen Arbeitsgang mit Wasser und optional einem Shampoo ausgewaschen werden. Da die erfindungsgemäß verwendeten Polysaccharide natürlich vorkommende Biopolymere darstellen, sind sie ohne Probleme in Kläranlagen biologisch abbaubar.

### Umformzusammensetzung (KR)

Die Umformzusammensetzung (KR) ist bevorzugt flüssig (0 bis 500 mPas bei 20°C), mittelviskos (> 400 bis 4000 mPas bei 20°C) oder cremeförmig (> 4000 bis 10.000 mPas bei 20 °C).

Die erfindungsgemäß verwendeten Umformzusammensetzungen (KR) enthalten mindestens eine Keratin reduzierende Verbindung, ausgewählt aus Thioglykolsäure und deren Salzen und/oder Thiomilchsäure und deren Salzen und/oder Cystein und/oder Acetylcystein und/oder Cysteamin und deren Salzen sowie Mischungen dieser Keratin reduzierenden Verbindungen. Bevorzugte erfindungsgemäß verwendete Umformzusammensetzungen (KR) sind dadurch gekennzeichnet, dass die mindestens eine Keratin reduzierende Verbindung in einer Gesamtmenge von 0,1 bis 20 Gew.%, bevorzugt 1 bis 15 Gew.-%, besonders bevorzugt 3 bis 13 Gew.-%, jeweils bezogen auf das Gewicht des Mittels (KR), enthalten ist. Außerordentlich bevorzugt sind Mittel (KR), die Thioglykolsäure und deren Salze und/oder Thiomilchsäure und deren Salze in einer Gesamtmenge von 5 bis 20 Gew.%, bevorzugt 7 bis 15 Gew.-%, besonders bevorzugt 10 bis 13 Gew.-%, jeweils bezogen auf das Gewicht des Mittels (KR), enthalten.

### Alkalisierungsmittel

Die Umformzusammensetzung (KR) enthält mindestens ein Alkalisierungsmittel, das ausgewählt ist aus Ammoniumhydroxid, Monoethanolamin und Ammoniumcarbonat sowie Mischungen hiervon. Bevorzugt enthält die Umformzusammensetzung (KR) Ammoniumhydroxid, also Ammoniak in Form seiner wässrigen Lösung. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt. In einer besonders bevorzugten Ausführungsform sind die erfindungsgemäß verwendeten Zusammensetzungen (KR) dadurch gekennzeichnet, dass sie Ammoniumhydroxid in einer Menge von 0,1 bis 5 Gew.-%, bevorzugt von 0,2 bis 3 Gew.-%, weiter bevorzugt von 0,3 bis 2 Gew.-% und besonders bevorzugt von 0,4 bis 1,5 Gew.-% - bezogen auf das Gewicht der erfindungsgemäß verwendeten Zusammensetzung (KR) - enthalten.

Zusätzlich zu bzw. anstelle von Ammoniumhydroxid enthalten bevorzugte erfindungsgemäß verwendete Zusammensetzungen (KR) Monoethanolamin.
Zur Erzielung einer maximalen Geruchsminimierung und zur Optimierung der Umformeigenschaften ist Monoethanolamin in einer Gesamtmenge von 0,1 - 6,0 Gew.-%, bevorzugt von 0,5 - 4 Gew.-%, weiter bevorzugt von 1 bis 3,0 Gew.-% und besonders bevorzugt von 2,0 bis 2,5 Gew.-% - bezogen auf das Gewicht der erfindungsgemäß verwendeten Zusammensetzung (KR) - enthalten.
Zusätzlich zu bzw. anstelle von Ammoniumhydroxid oder Monoethanolamin enthalten bevorzugte erfindungsgemäß verwendete Zusammensetzungen (KR) Ammoniumcarbonat.
Zur Erzielung einer maximalen Geruchsminimierung und zur Optimierung der Umformeigenschaften ist Ammoniumcarbonat in einer Gesamtmenge von 0,1 - 6,0 Gew.-%, bevorzugt von 0,5 - 4 Gew.-%, weiter bevorzugt von 1 bis 3,0 Gew.-% und besonders bevorzugt von 2,0 bis 2,5 Gew.-% - bezogen auf das Gewicht der erfindungsgemäß verwendeten Zusammensetzung (KR) - enthalten.

Die Fixierung der umgeformten Keratinfasern erfolgt erst durch den Einfluss eines Oxidationsmittels. Üblicherweise wird hierfür Wasserstoffperoxid verwendet. In einer bevorzugten Ausführungsform wird das Wasserstoffperoxid als wässrige Lösung verwendet. Erfindungsgemäß bevorzugte Oxidationsmittelzubereitungen sind dadurch gekennzeichnet, dass sie 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges H₂O₂) enthalten.
Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Natriumsilikate können als Alkalisierungsmittel für die Polysaccharid-Zusammensetzung (D) bevorzugt sein. Bei Natriumsilikaten im Sinne der vorliegenden Erfindung handelt es sich um chemische Verbindungen, die sich aus Natriumoxid und Siliciumdioxid zusammensetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel Na₄SiO₄, das auch als Natriumorthosilikat bezeichnet wird.
Weitere Beispiele für geeignete Natriumsilikate sind das Dinatriummetasilikat bzw. Natriummetasilikat mit der Summenformel Na₂SiO₃, das Dinatriumdisilikat mit der Summenformel Na₂Si₂O₅ oder das Dinatriumtrisilikat mit der Summenformel Na₂Si₃O₇.
Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser.

Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.
Die molare Zusammensetzung bei Wassergläsern beträgt üblicherweise 2 bis 4 mol SiO₂ auf 1 mol Alkalioxid (Na₂O).
Ein Beispiel für ein bevorzugtes Natriumsilikat ist Natronwasserglas, das in Form seiner wässrigen Lösung vorliegt, einen Na₂O-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat. Erfindungsgemäß bevorzugt verwendete Zusammensetzungen (D) enthalten als Alkalisierungsmittel mindestens ein Natriumsilikat, bevorzugt Natriummetasilikat oder Natronwasserglas, in einer Gesamtmenge von 0,1 bis 5 Gew.-%, bevorzugt 0,2 bis 3 Gew.-%, besonders bevorzugt 0,3 bis 2 Gew.-%, außerordentlich bevorzugt 0,5 bis 1,5 Gew.-%, jeweils bezogen auf das Gewicht der erfindungsgemäß verwendeten Zusammensetzung (D).
Weitere erfindungsgemäß bevorzugt verwendete Zusammensetzungen enthalten als Alkalisierungsmittel mindestens ein Alkalimetall- oder Erdalkalimetallcarbonat, das bevorzugt ausgewählt ist aus Natriumcarbonat, Kaliumcarbonat und Magnesiumcarbonat, sowie Mischungen dieser Carbonate. Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf das Gewicht der erfindungsgemäß verwendeten Zusammensetzung. Auch basische Aminosäuren, insbesondere Arginin, Lysin oder Histidin sowie Mischungen dieser Aminosäuren, insbesondere Mischungen aus Arginin und Lysin, können in bevorzugt verwendeten Zusammensetzungen als Alkalisierungsmittel enthalten sein, bevorzugt in einer Gesamtmenge von 0,01 bis 10 Gew.-%, bevorzugt 0,1 bis 5 Gew.-%, besonders bevorzugt 0,5 bis 3 Gew.-%, jeweils bezogen auf das Gewicht der erfindungsgemäß verwendeten Zusammensetzung.

### Konsistenzgeber

Erfindungsgemäß bevorzugt verwendete Zusammensetzungen (KR) enthalten mindestens einen Konsistenzgeber. Geeignete Konsistenzgeber sind insbesondere Fettsubstanzen mit einem Schmelzpunkt von 25 °C oder darüber bei 1013 mbar Umgebungsdruck sowie polymere Verdicker, die von den erfindungsgemäß verwendeten Polysacchariden, die mit Calciumionen in wässrigem Medium ein Gel bilden, verschieden sind. Derartige Konsistenzgeber sind bevorzugt ausgewählt aus linearen gesättigten 1-Alkanolen mit 12-30 Kohlenstoffatomen und Glycerylfettsäureestern der allgemeinen Formel (I), 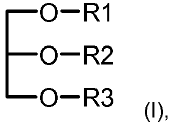 worin
R1, R2 und R3 unabhängig voneinander für ein Wasserstoffatom oder eine Gruppierung der Formel (II) stehen, 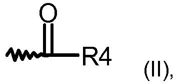 worin
R4 für eine unverzweigte oder verzweigte, gesättigte oder ungesättigte C₁₁-C₂₇-Alkylgruppe steht, mit der Maßgabe, dass mindestens einer und maximal zwei der Reste ausgewählt aus R1, R2 und R3 für eine Gruppierung der Formel (II) stehen.
Der Rest R4 in der Formel (II) steht für eine unverzweigte oder verzweigte, gesättigte oder ungesättigte C₁₁-C₂₇-Alkylgruppe.
Bevorzugt steht R4 für eine unverzweigte, gesättigte C₁₁-C₂₇-Alkylgruppe. Weiter bevorzugt steht R4 für eine unverzweigte, gesättigte C₁₃-C₂₃-Alkylgruppe. Besonders bevorzugt steht R4 für eine unverzweigte, gesättigte C₁₅-C₁₇-Alkylgruppe.
Erfindungsgemäß besonders bevorzugte Glycerylfettsäureester der allgemeinen Formel (I) sind ausgewählt aus mindestens einer Verbindung aus der Gruppe der Formeln (la) bis (Id):

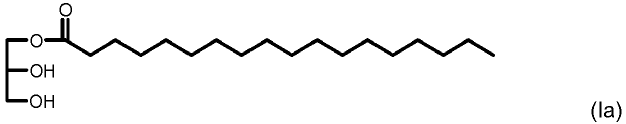

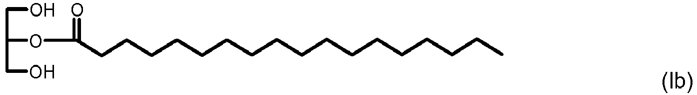

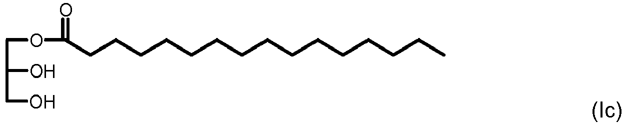

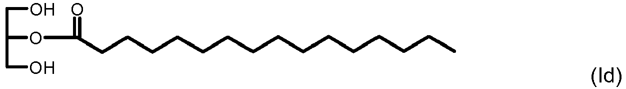

Die Verbindungen der Formeln (la) bis (Id) sind auch unter den Namen Glycerylmonostearat und Glycerylmonopalmitat bekannt.
Weitere erfindungsgemäß bevorzugt verwendete Umformzusammensetzungen (KR) sind dadurch gekennzeichnet, dass als Glycerylfettsäureester der allgemeinen Formel (I) mindestens eine Verbindung aus der Gruppe der Formeln (Ie) bis (Ih) enthalten ist:

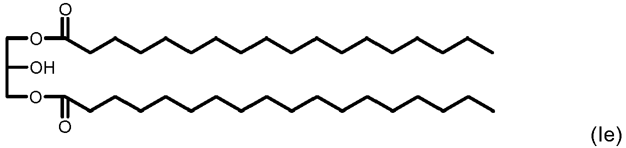

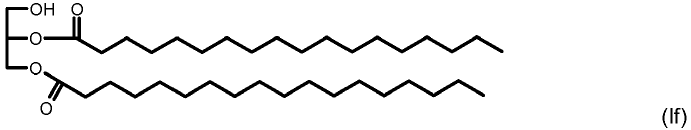

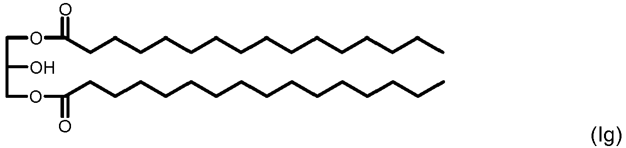

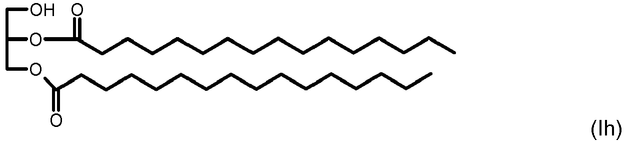

Die Verbindungen der Formel (Ie) bis (Ih) sind auch unter den Namen Glyceryldistearat und Glyceryldipalmitat bekannt.
Erfindungsgemäß bevorzugte lineare gesättigte 1-Alkanole mit 12 - 30 Kohlenstoffatomen sind ausgewählt aus Laurylalkohol, Myristylalkohol, Cetylalkohol, Stearylalkohol, Arachidylalkohol und Behenylalkohol sowie aus Mischungen dieser Alkanole, besonders bevorzugt aus Cetylalkohol, Stearylalkohol, Arachidylalkohol, Behenylalkohol und Cetylalkohol/ Stearylalkohol-Mischungen. Weitere erfindungsgemäß bevorzugte Konsistenzgeber sind Ethylenglycolmonosterat, Ethylenglycoldistearat, Wachse, Glycerintriester, die einen Schmelzpunkt von 25 °C oder darüber aufweisen, insbesondere hydriertes Rizinusöl (Castorwachs) und ethoxylierte hydrierte Rizinusöle, und Fettsäure-Fettalkoholester mit einem Schmelzpunkt von 25 °C oder darüber, sowie Mischungen dieser Substanzen.
Erfindungsgemäß bevorzugt verwendete Zusammensetzungen (KR) enthalten mindestens einen Fettsubstanz-Konsistenzgeber in einer Gesamtmenge von 0,1 bis 30 Gew.-%, bevorzugt 0,5 bis 20 Gew.-%, besonders bevorzugt 2 bis 15 Gew.-%, außerordentlich bevorzugt 3 bis 10 Gew.-%, jeweils bezogen auf das Gewicht der Zusammensetzung (KR). Weitere bevorzugte verwendete Zusammensetzungen (KR) enthalten, jeweils bezogen auf ihr Gewicht, mindestens ein lineares gesättigtes 1-Alkanol mit 12 - 30 Kohlenstoffatomen in einer Gesamtmenge von 0,1 bis 30 Gew.-%, bevorzugt 0,5 bis 20 Gew.-%, besonders bevorzugt 2 bis 15 Gew.-%, außerordentlich bevorzugt 3 bis 10 Gew.-%, jeweils bezogen auf das Gewicht der Zusammensetzung (KR).

In einer besonders bevorzugten Ausführungsform der Erfindung sind die erfindungsgemäß verwendeten Zusammensetzungen (KR), (C) und (D) und das Fixiermittel frei von anionischen oder kationischen Assoziativpolymeren, die mit ggf. ethoxylierten, C₆ - C₂₄-Alkyl- oder C₆ - C₂₄-AlkenylGruppen substituiert sind. In einer außerordentlich bevorzugten Ausführungsform der Erfindung sind die erfindungsgemäß verwendeten Zusammensetzungen KR), (C) und (D) und das Fixiermittel frei von anionischen oder kationischen Assoziativpolymeren, die mit ggf. ethoxylierten, C₆ - C₂₄-Alkyl- oder C₆ - C₂₄-Alkenyl-Gruppen substituiert sind und ausgewählt sind aus Acrylates/Ceteth-20 Itaconate Copolymeren, Polyurethane-39-Polymeren, Acrylates/Beheneth-25 Methacrylate Copolymeren und Acrylates/C10-30 Alkyl Acrylate Crosspolymeren.

### Gelierendes Metallsalz in Umformzusammensetzung (KR)

Sofern das mindestens eine Salz eines mehrwertigen Metallions, ausgewählt aus Calcium-, Strontium-, Barium- und Aluminiumsalzen mit einer Wasserlöslichkeit bei 20°C von mindestens 500 mg/l und unter bestimmten Bedingungen auch ausgewählt aus Kaliumsalzen mit einer Wasserlöslichkeit bei 20°C von mindestens 500 mg/l, in der Umformzusammensetzung (KR) enthalten ist, liegt es bevorzugt in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (KR), vor. Besonders bevorzugt enthält die Umformzusammensetzung (KR) mindestens ein gelbildendes Salz, das bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, aufweist. Das erfindungsgemäß außerordentlich bevorzugte Calciumchlorid hat eine Wasserlöslichkeit von 740 g/l bei 20°C. Andere erfindungsgemäß besonders bevorzugte gelbildende Salze sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein. Besonders bevorzugt enthält die Umformzusammensetzung (KR) mindestens ein Salz, ausgewählt aus Calciumchlorid, Calciumchlorid, Calciumlactat, Calciumgluconat und Calciumgluconatlactat sowie Mischungen hiervon in einer Gesamtmenge von 0,1 - 5 Gew.-%, besonders bevorzugt 0,2 - 3 Gew.-%, außerordentlich bevorzugt 0,5 - 2,5 Gew.-%, jeweils bezogen auf das Gewicht der Zusammensetzung (KR). Außerordentlich bevorzugt enthält die Umformlzusammensetzung (KR) 0,1 - 5 Gew.-%, besonders bevorzugt 0,2 - 3 Gew.-%, außerordentlich bevorzugt 0,5 - 2,5 Gew.-%, Calciumchlorid, jeweils bezogen auf das Gewicht der Zusammensetzung (KR).

### Organische Lösemittel

Weiterhin können alle erfindungsgemäß verwendeten Zusammensetzungen, insbesondere das Fixiermittel und die Umformzusammensetzungen (KR) mindestens ein organisches Lösemittel enthalten, das bevorzugt ausgewählt ist aus einem oder mehreren ein- oder mehrwertigen Alkoholen mit 2 bis 9 Kohlenstoffatomen, Polyethylenglycolen mit 2 bis 20 Ethylenglycoleinheiten sowie Mischungen dieser Lösemittel, bevorzugt in einer Gesamtmenge von 0,001 bis 80 Gew.-%, besonders bevorzugt 0,01 bis 50 Gew.-%, außerordentlich bevorzugt 0,5 bis 20 Gew.-%, weiter außerordentlich bevorzugt 1,5 bis 10 Gew.-%, jeweils bezogen auf das Gewicht der jeweiligen erfindungsgemäß verwendeten Zusammensetzung. Bevorzugte organische Lösemittel sind ausgewählt aus C₁-C₄-Alkoholen, insbesondere Ethanol und Isopropanol, weiterhin ausgewählt aus mehrwertigen Alkoholen mit 2 bis 9, bevorzugt ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, Dipropylenglycol, Tripropylenglycol, Diglycerin und Triglycerin sowie weiterhin ausgewählt aus Polyethylenglycolen mit 2 bis 20 Ethylenglycoleinheiten, insbesondere PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Die Verwendung von Mischungen der vorgenannten Substanzen ist erfindungsgemäß ebenfalls bevorzugt.

Um die Auswaschbarkeit der Umformzusammensetzung (KR) von den Keratinfasern zu begünstigen, ist es erfindungsgemäß bevorzugt, dass die Umformzusammensetzung (KR) mindestens ein Tensid enthält, bevorzugt in einer Gesamtmenge von 0,1 bis 15 Gew.-%, bevorzugt 1 bis 8 Gew.-%, bezogen auf das Gewicht der Umformzusammensetzung (KR).
Auch das Fixiermittel enthält bevorzugt mindestens ein Tensid, bevorzugt in einer Gesamtmenge von 0,1 bis 10 Gew.-%, bevorzugt 1 bis 5 Gew.-%, bezogen auf das Gewicht des Fixiermittels.
Geeignete Tenside sind nichtionische, anionische, kationische und amphotere Tenside. Bevorzugt sind nichtionische und anionische Tenside sowie Mischungen hiervon. Bevorzugte anionische Tenside sind Alkylsulfate, Alkylethersulfate und Ethercarbonsäuren mit 10 bis 18 C-Atomen in der Alkylgruppe und bis zu 12 Glykolethergruppen im Molekül. Besonders bevorzugte amphotere Tenside sind Cocamidopropylbetain, N-Kokosalkylaminopropionat, Kokosacylaminoethylaminopropionat und C₁₂-C₁₈-Acylsarcosin.
Bevorzugte nichtionische Tenside sind ausgewählt aus ethoxylierten C10-C24-Fettalkoholen mit einem Ethoxylierungsgrad von 4 bis 120, bevorzugt 10 bis 50, besonders bevorzugt 12 bis 20, wobei Mischungen dieser ethoxylierten C10-C24-Fettalkohole mit unterschiedlichen Ethoxylierungsgraden besonders bevorzugt sind. Weitere bevorzugte nichtionische Tenside sind ausgewählt aus ethoxylierten Glycerinmono-, -di- und -triestern von C10-C24-Fettsäuren, beispielsweise PEG-40 Castor Oil, PEG-40 Hydrogenated Castor Oil, PEG-60 Castor Oil oder PEG-60 Hydrogenated Castor Oil. Weitere bevorzugte nichtionische Tenside sind ausgewählt aus C₈ - C₂₂-Alkylmono- und - oligoglycosiden. Cs -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Handelsübliche Produkte, die zum Beispiel unter dem Warenzeichen Plantacare® erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon.
Bevorzugte kationische Tenside sind beispielsweise Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen.

### Gelierzusammensetzung (C)

Um erfindungsgemäß die Geruchs-Barriereschicht aus Polysaccharidgel in situ auf den Keratinfasern herzustellen, wird auf die mit der Umformzusammensetzung (KR) behandelten Keratinfasern eine Gelierzusammensetzung (C) appliziert, beispielsweise mit einem Pinsel oder ähnlichen Applikator, oder - was besonders bevorzugt ist - durch Aufsprühen, wobei die Gelierzusammensetzung (C) enthält:
c)i. 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l enthält,
c)ii. 50 - 99 Gew.-%, bezogen auf das Gewicht der Zusammensetzung (C), Wasser,
c)iii. optional mindestens ein Kationpolymer, das bevorzugt ausgewählt ist aus kationisierten Guar-Ethern;
c)iv. wobei die Gelierzusammensetzung (C) einen pH-Wert im Bereich von 4 bis 12, bevorzugt im Bereich von 4 - 9, besonders bevorzugt 6,5 - 8, aufweist, gemessen bei 20°C.

### Gel bildendes Salz / Wasserlöslichkeit

Das mindestens eine Salz c)i weist bei 20°C eine Wasserlöslichkeit von mindestens 500 mg/l, bevorzugt von mindestens 40 g/l, besonders bevorzugt von mindestens 200 g/l, außerordentlich bevorzugt von mindestens 350 g/l, auf. Das erfindungsgemäß außerordentlich bevorzugte Calciumchlorid hat eine Wasserlöslichkeit von 740 g/l bei 20°C. Andere erfindungsgemäß besonders bevorzugte Salze c)i sind ausgewählt aus Calciumacetat (Wasserlöslichkeit 374 g/l), Calciumlactat, Calciumgluconat, Calciumgluconatlactat. Ebenfalls bevorzugt sind Aluminiumchlorid, Aluminiumhydroxychlorid, Aluminiumsulfat, Aluminiumlactat, Aluminiumacetat, Aluminiumgluconat. Für die Gelierung von kappa-Carragheenan sind Kaliumchlorid, Kaliumacetat, Kaliumlactat, Kaliumgluconat und Kaliumsulfat bevorzugt, wobei Kaliumchlorid, Kaliumlactat, und Kaliumgluconat besonders bevorzugt sind. Geeignet sind weiterhin Strontiumchlorid und Bariumchlorid. Bevorzugt können auch Mischungen der vorgenannten Salze enthalten sein.
Die Gelierzusammensetzung (C) enthält, jeweils bezogen auf ihr Gewicht, 50 - 99 Gew.-%, bevorzugt 70 - 95 Gew.-%, besonders bevorzugt 75 - 92 Gew.-% Wasser.
Die Gelierzusammensetzung (C) weist einen pH-Wert im Bereich von 4 bis 12, bevorzugt im Bereich von 4 - 9, besonders bevorzugt 6,5 - 8, auf, gemessen bei 20°C. Ob ein saurer oder ein alkalischer pH-Wert eingestellt ist, hängt unter anderem davon ab, ob die Viskosität der Zusammensetzung (C) zur Verbesserung ihrer Applikationseigenschaften, insbesondere zur Verbesserung des Sprühbildes, mit einem Verdickungsmittel angehoben werden soll. Zur bevorzugt moderaten Andickung der Zusammensetzung (C) sind besonders gut kationische Polymere geeignet. Bevorzugte kationische Polymere sind ausgewählt aus kationischen Guarderivaten, insbesondere aus kationisierten Guar-Ethern, insbesondere aus Polymeren mit den INCI-Bezeichnungen Hydroxypropyl Guar Hydroxypropyltrimonium Chloride und Guar Hydroxypropyltrimonium Chloride. Sofern die Gelierzusammensetzung (C) ein kationisches Polymer enthält, ist dieses in einer Gesamtmenge von 0,01 - 1 Gew.-%, bevorzugt 0,1 bis 0,8 Gew.-%, besonders bevorzugt 0,2 bis 0,5 Gew.-%, jeweils bezogen auf das Gewicht der Zusammensetzung (C), enthalten. Sofern die Gelierzusammensetzung (C) ein kationisches Polymer enthält, weist sie bevorzugt einen pH-Wert im Bereich von 4 - 6,5 auf, gemessen bei 20°C.
Die Einstellung des pH-Wertes erfolgt, wie auch für die übrigen erfindungsgemäß verwendeten Zusammensetzungen, mit üblichen pH-Stellmitteln, wie insbesondere Citronensäure, Milchsäure, NaOH, KOH und ähnlichen kosmetisch verträglichen Säuren und Basen.
In einer bevorzugten Ausführungsform des erfindungsgemäßen Umformverfahrens wird die Gelierzusammensetzung (C) *in situ* oder erst 0,01 bis 24 Stunden vor Durchführung des erfindungsgemäßen Verfahrens durch Vermischen einer festen, bevorzugt pulverförmigen, Gelierzusammensetzung (C'), enthaltend das mindestens eine unter c)i genannte Salz in Pulverform und optional mindestens ein Kationpolymer, das bevorzugt ausgewählt ist aus kationisierten Guar-Ethern, in Pulverform, mit Wasser c) iii hergestellt. Besonders bevorzugt enthält die Gelierzusammensetzung (C') zusätzlich ein festes, bevorzugt pulverförmiges, pH-Stellmittel, z. B. eine Säure wie Citronensäure, und/oder ein Alkalisierungsmittel, wie Natriumsilikat.

### Polysaccharid-Zusammensetzung (D)

Eine besonders bevorzugte Ausführungsform des erfindungsgemäßen Umformverfahrens ist dadurch gekennzeichnet, dass das mindestens eine Polysaccharid, das mit Calciumionen in wässrigem Medium ein Gel bilden kann und das ausgewählt ist aus kappa-Carragheenan, iota-Carragheenan und Pektin, und/ oder mindestens einem Salz der vorgenannten Polysaccharide, das ausgewählt ist aus den Alkalimetall-, den Ammonium-, den Mono-, Di- und Trialkylammonium-, den Mono-, Di- und Trialkanolammonium- und den Magnesiumsalzen, wobei im Falle von Salzen des kappa-Carragheenans dessen Alkalimetall-Salz nur aus Lithium- und Natriumsalzen ausgewählt ist, bereitgestellt wird in Form einer Polysaccharid-Zusammensetzung (D), enthaltend
iii.1 mindestens ein Polysaccharid, das mit Calciumionen in wässrigem Medium ein Gel bilden kann und ausgewählt ist aus kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
iii.2 50 - 99,9, bevorzugt 60 bis 95, besonders bevorzugt 80 bis 90 Gew.-% Wasser, jeweils bezogen auf das Gewicht der Zusammensetzung (D),
iii.3 optional mindestens ein Alkalisierungsmittel, das bevorzugt ausgewählt ist aus pulverförmigen Natriumsilikaten, insbesondere pulverförmigen Natriummetasilikaten,
iii.4 optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),
iii.5 optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),
wobei optional die Polysaccharid-Zusammensetzung (D) durch Vermischen einer festen, bevorzugt pulverförmigen, Polysaccharid-Zusammensetzung (D'), enthaltend iii.1 und optional iii.3 und/oder optional iii.4, mit der Komponente iii.2 und optional mit der Komponente iii.5 *in situ* oder 0,01 bis 24 Stunden vor Durchführung des erfindungsgemäßen Umformverfahrens hergestellt wird.

In einer bevorzugten Ausführungsform des erfindungsgemäßen Umformverfahrens wird die Polysaccharid-Zusammensetzung (D) *in situ* oder 0,01 bis 24 Stunden vor Durchführung des erfindungsgemäßen Umformverfahrens durch Vermischen einer festen, bevorzugt pulverförmigen, Polysaccharid-Zusammensetzung (D'), enthaltend i.1 und optional iii.3 und/oder optional iii.4, mit der Komponente ii.2 und optional mit der Komponente ii.5 hergestellt.

Bei der in situ Herstellung der Polysaccharid-Zusammensetzung (D) hat es sich bewährt, das mindestens eine Polysaccharid, das mit Calciumionen in wässrigem Medium ein Gel bilden kann, in 25°C bis 40 °C warmes Wasser aufzulösen, das mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D), enthält. Das Öl sollte vor der Zugabe des Polysaccharids im Wasser enthalten sein. Dadurch wird die Auflösung des Polysaccharids deutlich begünstigt. Erfindungsgemäß außerordentlich bevorzugte Öle sind ausgewählt aus natürlichen und synthetischen Kohlenwasserstoffen, besonders bevorzugt aus Mineralölen, Paraffinölen, C₁₈-C₃₀-Isoparaffinen, insbesondere Isoeicosan, Polyisobutene und Polydecene, weiterhin ausgewählt aus C₈-C₁₆-Isoparaffinen, insbesondere aus Isodecan, Isododecan, Isotetradecan und Isohexadecan sowie Mischungen hiervon, sowie 1,3-Di-(2-ethylhexyl)-cyclohexan.

Weitere erfindungsgemäß bevorzugte Öle sind ausgewählt aus den Estern der linearen oder verzweigten gesättigten oder ungesättigten Fettalkohole mit 2-30 Kohlenstoffatomen mit linearen oder verzweigten gesättigten oder ungesättigten Fettsäuren mit 2-30 Kohlenstoffatomen, die hydroxyliert sein können. Dazu zählen Cetyl-2-ethylhexanoat, 2-Hexyldecylstearat (z. B. Eutanol® G 16 S), 2-Hexyldecyllaurat, Isodecylneopentanoat, Isononylisononanoat, 2-Ethylhexylpalmitat (z. B. Cegesoft® C 24) und 2-Ethylhexylstearat (z. B. Cetiol® 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte Öle sind ausgewählt aus Fettalkoholen mit 6-30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol (Eutanol® G 16), 2-Octyldodecanol (Eutanol® G), 2-Ethylhexylalkohol und Isostearylalkohol.

Weitere bevorzugte Öle sind ausgewählt aus Mischungen aus Guerbetalkoholen und Guerbetalkoholestern, z.B. dem Handelsprodukt Cetiol® PGL (2-Hexyldecanol und 2-Hexyldecyllaurat).

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318, Myritol® 331 (BASF) oder Miglyol® 812 (Hüls) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexyl-succinat und Di-(2-hexyldecyl)-succinat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß bevorzugte kosmetische Öle sind ausgewählt aus den C8-C22-Fettalkoholestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure. Solche Ester auf Basis von linearen C14/15-Alkanolen, z. B. C12-C15-Alkyllactat, und von in 2-Position verzweigten C12/13-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß bevorzugte kosmetische Öle sind ausgewählt aus den symmetrischen, unsymmetrischen oder cyclischen Estern der Kohlensäure mit C₃₋₂₂-Alkanolen, C₃₋₂₂-Alkandiolen oder C₃₋₂₂-Alkantriolen, z. B. Dicaprylylcarbonat (Cetiol® CC) oder die Ester gemäß der Lehre der DE19756454 A1, insbesondere Glycerincarbonat.

Weitere kosmetische Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/ oder L₄, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt. Es kann erfindungsgemäß außerordentlich bevorzugt sein, Mischungen der vorgenannten Öle einzusetzen.

Auch durch Zugabe eines Tensids, zum Beispiel in einer Gesamtmenge von 0,05 bis 3 Gew.-%, bevorzugt 0,1 bis 1 Gew.-%, besonders bevorzugt 0,3 bis 0,8 Gew.-%, bezogen auf das Gewicht der Zusammensetzung (D), kann die Auflösung des Polysaccharids günstig beeinflusst werden. Allerdings kann ein Tensidgehalt bewirken, dass die Polysaccharid-Zusammensetzung (D), die zum Lösen des Polysaccharids geschüttelt werden sollte, zu stark schäumt, was ihre Applikation erschwert. Bevorzugt enthält die Polysaccharid-Zusammensetzung (D) daher kein Tensid.

Besonders bevorzugt verwendete Polysaccharid-Zusammensetzungen (D) und (D') sind dadurch gekennzeichnet, dass das mindestens eine Polysaccharid, das mit Calciumionen in wässrigem Medium ein Gel bilden kann, ausgewählt ist aus kappa-Carragheenan, das Lithiumsalz von kappa-Carragheenan, das Natriumsalz von kappa-Carragheenan, iota-Carragheenan, das Lithiumsalz von iota-Carragheenan, das Natriumsalz von iota-Carragheenan, das Kaliumsalz von iota-Carragheenan, das Ammoniumsalz von iota-Carragheenan, Pektin, Natriumpektinat, Ammoniumpektinat, Kaliumpektinat und Magnesiumpektinat, sowie Mischungen dieser Substanzen.

Es ist erfindungsgemäß bevorzugt, dass die Polysaccharid-Zusammensetzung (D) einen pH-Wert im Bereich von 7 bis 12, bevorzugt 8 bis 10, besonders bevorzugt 8,5 bis 9,5, aufweist, jeweils bei 20°C gemessen. Daher enthält die Polysaccharid-Zusammensetzung (D) bevorzugt mindestens ein Alkalisierungsmittel, das besonders bevorzugt ausgewählt ist aus pulverförmigen Natriumsilikaten, insbesondere pulverförmigen Natriummetasilikaten. Aber auch die anderen vorstehend aufgeführten Alkalisierungsmittel können zur Einstellung des pH-Werts der Polysaccharid-Zusammensetzung (D) eingesetzt werden. Für die Polysaccharid-Zusammensetzung (D') ist ein Gehalt an mindestens einem festen, bevorzugt pulverförmigen, Alkalisierungsmittel bevorzugt, das bevorzugt ausgewählt ist aus pulverförmigen Natriumsilikaten, insbesondere pulverförmigen Natriummetasilikaten.

Es kann erfindungsgemäß bevorzugt sein, dass die Polysaccharid-Zusammensetzung (D) oder die Polysaccharid-Zusammensetzung (D') mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, enthält, bevorzugt in einer Gesamtmenge von 0,05 - 3 Gew.-%, bevorzugt 0,2 - 0,5 Gew.-%, bezogen auf das Gewicht der Zusammensetzung (D). Der Zusatz dieser Polymere dient vor allem dazu, die Löslichkeit des Polysaccharids bei der in situ-Herstellung der Zusammensetzung (D) zu verbessern. Bevorzugt ist das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, ausgewählt aus vernetzten Homopolymeren der Acrylsäure, vernetzten Homopolymeren der 2-Acrylamido-2-methylpropansulfonsäure, vernetzten Copolymeren bestehend aus Acrylsäure- und 2-Acrylamido-2-methylpropansulfonsäure-Einheiten, vernetzten Copolymeren bestehend aus 2-Methyl-2[(1-oxo-2-propenyl)amino]-1-propansulfonsäure-Einheiten und Hydroxyethylacrylat-Einheiten, vernetzten Copolymeren bestehend aus Acrylamid- und 2-Acrylamido-2-methylpropansulfonsäure-Einheiten, vernetzten Copolymeren bestehend aus Methacrylsäure-Einheiten und C1-C4-Alkylacrylat-Einheiten, vernetzten Copolymeren bestehend aus Acrylsäure-Einheiten und C1-C4-Alkylmethacrylat-Einheiten, sowie Mischungen hiervon.

Besonders bevorzugt weist das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, keine Monomere auf, die mit ggf. ethoxylierten, C₆ - C₂₄-Alkyl- oder C₆ - C₂₄-AlkenylGruppen substituiert sind.

## Patentansprüche

1. Verfahren zur permanenten Umformung, insbesondere zum Glätten oder zum Dauerwellen, von keratinischen Fasern, **gekennzeichnet durch** folgende Verfahrensschritte:
(i) Verformen der Fasern unter Zuhilfenahme von mechanischen Verformungshilfsmitteln, wie Wickler oder Papilloten, nach, vor oder während des Schritts (ii),
(ii) Auftragen einer chemischen Umformzusammensetzung (KR) zum Glätten oder zum Dauerwellen auf die Fasern, enthaltend
- mindestens eine Keratin reduzierende Verbindung, ausgewählt aus Thioglykolsäure und deren Salzen und / oder Thiomilchsäure und deren Salzen und/oder Cystein und/oder Acetylcystein und/oder Cysteamin und deren Salzen sowie Mischungen dieser Keratin reduzierenden Verbindungen, weiterhin
- Wasser, weiterhin
- mindestens ein Alkalisierungsmittel,
anschließend
(iii) Auftragen einer Polysaccharid-Zusammensetzung (D), enthaltend
iii.1 mindestens ein Polysaccharid, das mit Calciumionen in wässrigem Medium ein Gel bilden kann und ausgewählt ist aus kappa-Carragheenan, iota-Carragheenan und Pektin, und/oder mindestens einem Salz der vorgenannten Polysaccharide, ausgewählt aus den Alkalimetall-, den Ammonium-, den Mono-, Di- und Trialkylammonium-, den Mono-, Di- und Trialkanolammonium- und den Magnesiumsalzen, bevorzugt in einer Gesamtmenge von 0,1 - 5 Gew.-%, bevorzugt 0,5 - 2 Gew.-%, besonders bevorzugt 0,5 - 2,5 Gew.-%, jeweils bezogen auf das Gewicht der Zusammensetzung (D), wobei im Falle von Salzen des kappa-Carragheenans dessen Alkalimetall-Salz nur aus Lithium- und Natriumsalzen ausgewählt ist,
iii.2 50 - 99,9, bevorzugt 60 bis 95, besonders bevorzugt 80 bis 90 Gew.-% Wasser, jeweils bezogen auf das Gewicht der Zusammensetzung (D),
iii.3 optional mindestens ein Alkalisierungsmittel, das bevorzugt ausgewählt ist aus pulverförmigen Natriumsilikaten, insbesondere pulverförmigen Natriummetasilikaten,
iii.4 optional mindestens ein Polymer, enthaltend (Meth)Acrylsäure-, (Meth)Acrylsäureester-, (Meth)Acrylamid- und/oder 2-Acrylamido-2-methylpropansulfonsäure-Monomere, bevorzugt in einer Gesamtmenge von 0,05 - 3 Gew.-%, bevorzugt 0,2 - 0,5 Gew.-%, bezogen auf das Gewicht der Zusammensetzung (D),
iii.5. optional mindestens ein Öl in einer Gesamtmenge von 0,1 bis 10 Gew.-%, bevorzugt 0,3 bis 5 Gew.-%, besonders bevorzugt 1 bis 3 Gew.-%, jeweils bezogen auf das Gewicht der Polysaccharid-Zusammensetzung (D),
wobei optional die Polysaccharid-Zusammensetzung (D) durch Vermischen einer festen, bevorzugt pulverförmigen, Polysaccharid-Zusammensetzung (D'), enthaltend iii.1 und optional iii.3 und/oder optional iii.4, mit der Komponente iii.2 und optional mit der Komponente iii.5 *in situ* oder 0,01 bis 24 Stunden vor Durchführung des erfindungsgemäßen Glättungs- oder Umformverfahrens hergestellt wird,
(iv) Spülen der Fasern nach einer Einwirkzeit Z1, bevorzugt nach 5-60 Minuten, besonders bevorzugt nach 10-30 Minuten und gegebenenfalls Trocknen,
(v) anschließend Auftragen eines Fixiermittels, enthaltend mindestens ein Oxidationsmittel, das bevorzugt ausgewählt ist aus Wasserstoffperoxid, auf die Fasern und Abspülen nach einer Einwirkzeit Z2, bevorzugt nach 1-30 Minuten, besonders bevorzugt nach 5-20 Minuten,
**dadurch gekennzeichnet, dass**
in einer ersten Variante das Verfahren die Applikation von mindestens einem Salz eines mehrwertigen Metallions, ausgewählt aus Calcium-, Strontium-, Barium- und Aluminiumsalzen mit einer Wasserlöslichkeit bei 20°C von mindestens 500 mg/l, umfasst,
oder in einer zweiten Variante für den Fall, dass das vorgenannte Polysaccharid in der Polysaccharid-Zusammensetzung (D), das mit Calciumionen ein Gel bilden kann, kappa-Carragheenan oder eines seiner Salze umfasst, das Verfahren die Applikation von mindestens einem Salz, ausgewählt aus Kalium-, Calcium-, Strontium-, Barium- und Aluminiumsalzen mit einer Wasserlöslichkeit bei 20°C von mindestens 500 mg/l enthält umfasst,
wobei in beiden Varianten diese Calcium-, Strontium-, Barium- und Aluminiumsalze (1. Variante) bzw. Kalium-, Calcium-, Strontium-, Barium- und Aluminiumsalze (2. Variante) nicht in der Polysaccharid-Zusammensetzung (D) enthalten sind.

2. Verfahren gemäß Anspruch 1, **dadurch gekennzeichnet, dass** die Zusammensetzung (KR) 0,1 - 3 Gew.-%, bevorzugt 0,2 - 2 Gew.-%, besonders bevorzugt 0,4 - 1 Gew.-%, bezogen auf das Gewicht der Zusammensetzung (KR), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, enthält, sowie für den Fall, dass das mit Calciumionen Gel bildende Polysaccharid kappa-Carragheenan oder eines seiner Salze umfasst, das Mittel zum Glätten oder zur permanenten Umformung 0,1 - 3 Gew.-%, bevorzugt 0,2 - 2 Gew.-%, besonders bevorzugt 0,4 - 1 Gew.-%, jeweils bezogen auf das Gewicht der Zusammensetzung (KR), mindestens eines Salzes, ausgewählt aus Kalium-, Calcium-, Strontium-, Barium- und Aluminiumsalzen mit einer Wasserlöslichkeit bei 20°C von mindestens 500 mg/l enthält.

3. Verfahren gemäß einem der Ansprüche 1 oder 2, **dadurch gekennzeichnet, dass** das mindestens eine Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, das mindestens eine Salz eines mehrwertigen Metallions, ausgewählt aus Kalium-, Calcium-, Strontium-, Barium- und Aluminiumsalzen mit einer Wasserlöslichkeit bei 20°C von mindestens 500 mg/l, in das Verfahren eingebracht werden, indem es
A. der Umformzusammensetzung (KR) bereits bei der Herstellung zugesetzt wird, oder
B. pulverförmig bereitgestellt wird und der Umformzusammensetzung (KR) *in situ* zugesetzt wird, oder
C. in Form einer Wasser-basierten Gelierzusammensetzung (C) bereitgestellt und der Umformzusammensetzung (KR) *in situ* zugesetzt wird, oder
D. in Form einer Wasser-basierten Gelierzusammensetzung (C) bereitgestellt und nach dem Auftragen der Umformzusammensetzung (KR) auf die Keratinfasern ebenfalls auf die Keratinfasern aufgetragen, insbesondere aufgesprüht, wird.

4. Verfahren gemäß einem der Ansprüche 1 bis 3, **dadurch gekennzeichnet, dass** das mindestens eine Salz eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, das mindestens eine Salz eines mehrwertigen Metallions, ausgewählt aus Kalium-, Calcium-, Strontium-, Barium- und Aluminiumsalzen mit einer Wasserlöslichkeit bei 20°C von mindestens 500 mg/l, bereitgestellt wird in Form einer Gelierzusammensetzung (C), enthaltend
d)i. 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes eines mehrwertigen Metallions, ausgewählt aus Calcium-, Strontium-, Barium- und Aluminiumsalzen mit einer Wasserlöslichkeit bei 20°C von mindestens 500 mg/l, sowie für den Fall, dass das mit Calciumionen Gel bildende Polysaccharid kappa-Carragheenan oder eines seiner Salze umfasst, die Gelierzusammensetzung (C) 0,1 - 3 Gew.-%, bevorzugt 0,2 - 2 Gew.-%, besonders bevorzugt 0,4 - 1 Gew.-%, bezogen auf das Gewicht der Zusammensetzung (C), mindestens eines Salzes, ausgewählt aus Kalium-, Calcium-, Strontium-, Barium- und Aluminiumsalzen mit einer Wasserlöslichkeit bei 20°C von mindestens 500 mg/l enthält,
d)ii. 50 - 99 Gew.-%, bezogen auf das Gewicht der Zusammensetzung (C), Wasser,
d)iii. optional mindestens ein Kationpolymer, das bevorzugt ausgewählt ist aus kationisierten Guar-Ethern;
d)iv. wobei die Gelierzusammensetzung (C) einen pH-Wert im Bereich von 4 bis 12, bevorzugt im Bereich von 4 - 9, besonders bevorzugt 6,5 - 8, aufweist, gemessen bei 20°C;
wobei optional die Gelierzusammensetzung (C) *in situ* oder 0,01 bis 24 Stunden vor Durchführung des erfindungsgemäßen Umformverfahrens durch Vermischen einer festen, bevorzugt pulverförmigen, Gelierzusammensetzung (C'), enthaltend das mindestens eine unter d)i genannte Salz in Pulverform und optional mindestens ein Kationpolymer, das bevorzugt ausgewählt ist aus kationisierten Guar-Ethern, in Pulverform, mit Wasser d) iii hergestellt wird,
**dadurch gekennzeichnet, dass** die Gelierzusammensetzung (C) in einem Verfahrensschritt
- nach dem Applikationsschritt ii und vor dem Applizieren, bevorzugt Aufsprühen, der Polysaccharid-Zusammensetzung (D) auf die mit der Umformzusammensetzung (KR) beaufschlagten keratinischen Fasern appliziert, bevorzugt aufgesprüht, wird, oder
- vor dem Applikationsschritt ii zur Umformzusammensetzung (KR) gegeben wird oder
- nach dem Applizieren, bevorzugt Aufsprühen, der Polysaccharid-Zusammensetzung (D) auf die mit der Umformzusammensetzung (KR) beaufschlagten keratinischen Fasern appliziert, bevorzugt aufgesprüht, wird.

5. Verfahren gemäß Anspruch 3 oder 4, **dadurch gekennzeichnet, dass** das Gewichtsverhältnis von Gelierzusammensetzung (C) zur Umformzusammensetzung (KR) im Bereich von 1 : 20 bis 1:2, bevorzugt im Bereich von 1:10 bis 1:5 liegt.

6. Verfahren gemäß einem der Ansprüche 1 - 5, **dadurch gekennzeichnet, dass** das Gewichtsverhältnis von Polysaccharid-Zusammensetzung (D) zur Umformzusammensetzung (KR) im Bereich von 1 : 20 bis 1:2, bevorzugt im Bereich von 1:10 bis 1:5 liegt.

7. Verfahren gemäß einem der Ansprüche 3 - 6, **dadurch gekennzeichnet, dass** das Gewichtsverhältnis von Polysaccharid-Zusammensetzung (D) zur Gelierzusammensetzung (C) im Bereich von 0,2 : 1 bis 2:1, bevorzugt im Bereich von 0,5 : 1 bis 1,5 : 1 liegt und besonders bevorzugt 1:1 beträgt.

8. Verfahren gemäß einem der Ansprüche 1 - 7, **dadurch gekennzeichnet, dass** das mindestens eine Polymer, enthaltend (Meth)Acrylsäure-, (Meth)Acrylsäureester-, (Meth)Acrylamid- und/oder 2-Acrylamido-2-methylpropansulfonsäure-Monomere, ausgewählt ist aus vernetzten Homopolymeren der Acrylsäure, vernetzten Homopolymeren der 2-Acrylamido-2-methylpropansulfonsäure, vernetzten Copolymeren bestehend aus Acrylsäure- und 2-Acrylamido-2-methylpropansulfonsäure-Einheiten, vernetzten Copolymeren bestehend aus 2-Methyl-2[(1-oxo-2-propenyl)amino]-1-propansulfonsäure-Einheiten und Hydroxyethylacrylat-Einheiten, vernetzten Copolymeren bestehend aus Acrylamid- und 2-Acrylamido-2-methylpropansulfonsäure-Einheiten, vernetzten Copolymeren bestehend aus Methacrylsäure-Einheiten und C1-C4-Alkylacrylat-Einheiten, vernetzten Copolymeren bestehend aus Acrylsäure-Einheiten und C1-C4-Alkylmethacrylat-Einheiten, sowie Mischungen hiervon.

## Claims

1. A method for permanently shaping, in particular for straightening or perming, keratin fibers, **characterized by** the following method steps:
(i) shaping the fibers using mechanical shaping aids such as curlers or papillotes before, during or after step (ii),
(ii) applying a chemical shaping composition (KR) to the fibers, which composition is for smoothing or perming and contains
- at least one keratin-reducing compound selected from thioglycolic acid and the salts thereof and/or thiolactic acid and the salts thereof and/or cysteine and/or acetylcysteine and/or cysteamine and the salts thereof and mixtures of these keratin-reducing compounds, and furthermore
- water, and furthermore
- at least one alkalizing agent,
then
(iii) applying a polysaccharide composition (D) containing
iii.1 at least one polysaccharide that can form a gel with calcium ions in an aqueous medium and is selected from kappa-carrageenan, iota-carrageenan and pectin, and/or at least one salt of the aforementioned polysaccharides, selected from the alkali metal, ammonium, mono-, di- and trialkylammonium, mono-, di- and trialkanolammonium and magnesium salts, preferably in a total amount of 0.1 to 5 wt.%, more preferably 0.5 to 2 wt.%, particularly preferably 0.5 to 2.5 wt.%, in each case based on the weight of the composition (D), in the case of salts of kappa-carrageenan the alkali metal salt thereof only being selected from lithium and sodium salts,
iii.2 50 to 99.9, preferably 60 to 95, particularly preferably 80 to 90 wt.% water, in each case based on the weight of the composition (D),
iii.3 optionally at least one alkalizing agent, which is preferably selected from powdered sodium silicates, in particular powdered sodium metasilicates,
iii.4 optionally at least one polymer containing (meth)acrylic acid monomers, (meth)acrylic acid ester monomers, (meth)acrylamide monomers and/or 2-acrylamido-2-methylpropane sulfonic acid monomers, preferably in a total amount of 0.05 to 3 wt.%, preferably 0.2 to 0.5 wt.%, based on the weight of the composition (D),
iii.5 optionally at least one oil in a total amount of 0.1 to 10 wt.%, preferably 0.3 to 5 wt.%, particularly preferably 1 to 3 wt.%, in each case based on the weight of the polysaccharide composition (D),
the polysaccharide composition (D) optionally being prepared by mixing a solid, preferably powdered, polysaccharide composition (D') containing iii.1 and optionally iii.3 and/or optionally iii.4 with component iii.2 and optionally with component iii.5 *in situ* or 0.01 to 24 hours before the straightening or shaping method according to the invention is carried out,
(iv) rinsing the fibers after a contact time Z1, preferably after 5 to 60 minutes, particularly preferably after 10 to 30 minutes, and optionally drying said fibers,
(v) then applying a fixing agent to the fibers that contains at least one oxidizing agent which is preferably selected from hydrogen peroxide, and rinsing after a contact time Z2, preferably after 1 to 30 minutes, particularly preferably after 5 to 20 minutes,
**characterized in that**,
in a first variant, the method comprises the application of at least one salt of a polyvalent metal ion, selected from calcium, strontium, barium and aluminum salts having a water solubility at 20 °C of at least 500 mg/l,
or, in a second variant in the case that the aforementioned polysaccharide in the polysaccharide composition (D), which polysaccharide can form a gel with calcium ions, comprises kappa-carrageenan or one of the salts thereof, the method comprises the application of at least one salt selected from potassium, calcium, strontium, barium and aluminum salts having a water solubility at 20 °C of at least 500 mg/l,
said calcium, strontium, barium and aluminum salts (1st variant) or potassium, calcium, strontium, barium and aluminum salts (2nd variant) not being contained in the polysaccharide composition (D) in both variants.

2. The method according to claim 1, **characterized in that** the composition (KR) contains, based on the weight of the composition (KR), 0.1 to 3 wt.%, preferably 0.2 to 2 wt.%, particularly preferably 0.4 to 1 wt.%, of at least one salt of a polyvalent metal ion, selected from calcium, strontium, barium and aluminum salts having a water solubility at 20 °C of at least 500 mg/l, and, in the case that the polysaccharide which forms a gel with calcium ions comprises kappa-carrageenan or one of the salts thereof, the agent for straightening or for permanently shaping contains, in each case based on the weight of the composition (KR), 0.1 to 3 wt.%, preferably 0.2 to 2 wt.%, particularly preferably 0.4 to 1 wt.%, of at least one salt selected from potassium, calcium, strontium, barium and aluminum salts having a water solubility at 20 °C of at least 500 mg/l.

3. The method according to one of claims 1 or 2, **characterized in that** the at least one salt of a polyvalent metal ion, selected from calcium, strontium, barium and aluminum salts having a water solubility at 20 °C of at least 500 mg/l, and, in the case that the polysaccharide which forms a gel with calcium ions comprises kappa-carrageenan or one of the salts thereof, the at least one salt of a polyvalent metal ion, selected from potassium, calcium, strontium, barium and aluminum salts having a water solubility at 20 °C of at least 500 mg/l, are introduced into the method by
A. already being added to the shaping composition (KR) during manufacture, or
B. being provided in powder form and added to the shaping composition (KR) *in situ,* or
C. being provided in the form of a water-based gelling composition (C) and added to the shaping composition (KR) *in situ,* or
D. being provided in the form of a water-based gelling composition (C) and, after the shaping composition (KR) has been applied to the keratin fibers, likewise being applied, in particular sprayed, onto the keratin fibers.

4. The method according to one of claims 1 to 3, **characterized in that** the at least one salt of a polyvalent metal ion, selected from calcium, strontium, barium and aluminum salts having a water solubility at 20 °C of at least 500 mg/l, and, in the case that the polysaccharide which forms a gel with calcium ions comprises kappa-carrageenan or one of the salts thereof, the at least one salt of a polyvalent metal ion, selected from potassium, calcium, strontium, barium and aluminum salts having a water solubility at 20 °C of at least 500 mg/l, are provided in the form of a gelling composition (C) containing
d)i. 0.1 to 3 wt.%, preferably 0.2 to 2 wt.%, particularly preferably 0.4 to 1 wt.%, based on the weight of the composition (C), of at least one salt of a polyvalent metal ion, selected from calcium, strontium, barium and aluminum salts having a water solubility at 20 °C of at least 500 mg/l, and, in the case that the polysaccharide which forms a gel with calcium ions comprises kappa-carrageenan or one of the salts thereof, the gelling composition (C) contains 0.1 to 3 wt.%, preferably 0.2 to 2 wt.%, particularly preferably 0.4 to 1 wt.%, based on the weight of the composition (C), of at least one salt selected from potassium, calcium, strontium, barium and aluminum salts having a water solubility at 20 °C of at least 500 mg/l,
d)ii. 50 to 99 wt.% water, based on the weight of the composition (C),
d)iii. optionally at least one cation polymer which is preferably selected from cationized guar ethers;
d)iv. the gelling composition (C) having a pH in the range from 4 to 12, preferably in the range from 4 to 9, particularly preferably 6.5 to 8, measured at 20 °C;
the gelling composition (C) optionally being prepared *in situ* or 0.01 to 24 hours before the shaping method according to the invention is carried out, by mixing a solid, preferably powdered, gelling composition (C') containing the at least one salt mentioned in d)i in powder form and optionally containing at least one cation polymer in powder form, which is preferably selected from cationized guar ethers, with water d)iii,
**characterized in that**, in one method step, the gelling composition (C)
- is applied, preferably sprayed, onto the keratin fibers, which have been subjected to the shaping composition (KR), after application step ii and before the application, preferably spraying, of the polysaccharide composition (D), or
- is added to the shaping composition (KR) before application step ii, or
- is applied, preferably sprayed, onto the keratin fibers, which have been subjected to the shaping composition (KR), after the application, preferably spraying, of the polysaccharide composition (D).

5. The method according to claim 3 or 4, **characterized in that** the weight ratio of gelling composition (C) to shaping composition (KR) is in the range of 1:20 to 1:2, preferably in the range of 1:10 to 1:5.

6. The method according to one of claims 1-5, **characterized in that** the weight ratio of polysaccharide composition (D) to shaping composition (KR) is in the range of 1:20 to 1:2, preferably in the range of 1:10 to 1:5.

7. The method according to one of claims 3-6, **characterized in that** the weight ratio of polysaccharide composition (D) to gelling composition (C) is in the range of 0.2:1 to 2:1, preferably in the range of 0.5:1 to 1.5:1 and particularly preferably is 1:1.

8. The method according to one of claims 1-7, **characterized in that** the at least one polymer containing (meth)acrylic acid monomers, (meth)acrylic acid ester monomers, (meth)acrylamide monomers and/or 2-acrylamido-2-methylpropane sulfonic acid monomers is selected from crosslinked homopolymers of acrylic acid, cross-linked homopolymers of 2-acrylamido-2-methylpropane sulfonic acid, crosslinked copolymers consisting of acrylic acid units and 2-acrylamido-2-methylpropane sulfonic acid units, crosslinked copolymers consisting of 2-methyl-2[(1-oxo-2-propenyl)amino]-1-propane sulfonic acid units and hydroxyethyl acrylate units, crosslinked copolymers consisting of acrylamide units and 2-acrylamido-2-methylpropane sulfonic acid units, crosslinked copolymers consisting of methacrylic acid units and C1-C4 alkyl acrylate units, crosslinked copolymers consisting of acrylic acid units and C1-C4 alkyl methacrylate units, and mixtures thereof.

## Revendications

1. Procédé de mise en forme permanente, notamment de lissage ou d'ondulation permanente, de fibres kératiniques, **caractérisé par** les étapes de procédé suivantes :
(i) mise en forme des fibres à l'aide de moyens mécaniques d'aide à la mise en forme, tels que des bigoudis ou des papillotes, après, avant ou pendant l'étape (ii),
(ii) application sur les fibres d'une composition chimique de mise en forme (KR) destinée au lissage ou à l'ondulation permanente, contenant
- au moins un composé kératoréducteur choisi parmi l'acide thioglycolique et ses sels et/ou l'acide thiolactique et ses sels et/ou la cystéine et/ou l'acétylcystéine et/ou la cystéamine et leurs sels, ainsi que des mélanges de ces composés kératoréducteurs, et
- de l'eau, et
- au moins un agent alcalinisant,
puis
(iii) application d'une composition de polysaccharide (D) contenant
iii.1 au moins un polysaccharide pouvant former un gel avec des ions calcium en milieu aqueux et choisi parmi le kappa-carraghénane, l'iota-carraghénane et la pectine, et/ou au moins un sel des polysaccharides précités, choisi parmi les sels de métal alcalin, d'ammonium, de mono-, di- et trialkylammonium, de mono-, di- et trialkanolammonium et de magnésium, de préférence en une quantité totale de 0,1 à 5 % en poids, de préférence 0,5 à 2 % en poids, de manière particulièrement préférée 0,5 à 2,5 % en poids, respectivement rapporté au poids de la composition (D), dans le cas de sels du kappa-carraghénane, son sel de métal alcalin étant choisi uniquement parmi les sels de lithium et de sodium,
iii.2 50 à 99,9 % en poids, de préférence 60 à 95 % en poids, de manière particulièrement préférée 80 à 90 % en poids d'eau, respectivement rapporté au poids de la composition (D),
iii.3 éventuellement au moins un agent alcalinisant, de préférence choisi parmi les silicates de sodium en poudre, notamment les métasilicates de sodium en poudre,
iii.4 éventuellement au moins un polymère contenant des monomères d'acide (méth)acrylique, d'ester d'acide (méth)acrylique, de (méth)acrylamide et/ou d'acide 2-acrylamido-2-méthylpropanesulfonique, de préférence en une quantité totale de 0,05 à 3 % en poids, de préférence 0,2 à 0,5 % en poids, rapporté au poids de la composition (D),
iii.5 éventuellement au moins une huile en une quantité totale de 0,1 à 10 % en poids, de préférence 0,3 à 5 % en poids, de manière particulièrement préférée 1 à 3 % en poids, respectivement rapporté au poids de la composition de polysaccharide (D),
éventuellement la composition de polysaccharide (D) étant préparée en mélangeant une composition de polysaccharide solide, de préférence en poudre (D') contenant iii.1 et éventuellement iii.3 et/ou éventuellement iii.4, avec le constituant iii.2 et éventuellement avec le constituant iii.5 *in situ* ou 0,01 à 24 heures avant la mise en œuvre du procédé de lissage ou de mise en forme selon l'invention,
(iv) rinçage des fibres après un temps de pose Z1, de préférence après 5 à 60 minutes, de manière particulièrement préférée après 10 à 30 minutes et, éventuellement, séchage,
(v) puis application sur les fibres d'un agent fixant contenant au moins un agent oxydant, de préférence choisi parmi le peroxyde d'hydrogène, et rinçage après un temps de pose Z2, de préférence après 1 à 30 minutes, de manière particulièrement préférée après 5 à 20 minutes,
**caractérisé**
**en ce que**, dans une première variante, le procédé comprend l'application d'au moins un sel d'un ion métallique polyvalent choisi parmi les sels de calcium, de strontium, de baryum et d'aluminium avec une solubilité dans l'eau à 20 °C d'au moins 500 mg/l,
ou **en ce que**, dans une seconde variante dans le cas où le polysaccharide précité dans la composition de polysaccharide (D), qui peut former un gel avec des ions calcium, comprend du kappa-carraghénane ou l'un de ses sels, le procédé comprend l'application d'au moins un sel choisi parmi les sels de potassium, de calcium, de strontium, de baryum et d'aluminium avec une solubilité dans l'eau à 20 °C d'au moins 500 mg/l,
dans les deux variantes, ces sels de calcium, de strontium, de baryum et d'aluminium (1ère variante) ou ces sels de potassium, de calcium, de strontium, de baryum et d'aluminium (2ème variante) n'étant pas contenus dans la composition de polysaccharide (D).

2. Procédé selon la revendication 1, **caractérisé en ce que** la composition (KR) contient 0,1 à 3 % en poids, de préférence 0,2 à 2 % en poids, de manière particulièrement préférée 0,4 à 1 % en poids, rapporté au poids de la composition (KR), d'au moins un sel d'un ion métallique polyvalent choisi parmi les sels de calcium, de strontium, de baryum et d'aluminium avec une solubilité dans l'eau à 20 °C d'au moins 500 mg/l, ainsi que, dans le cas où le polysaccharide formant un gel avec des ions calcium comprend du kappa-carraghénane ou l'un de ses sels, **en ce que** l'agent de lissage ou de mise en forme permanente contient 0,1 à 3 % en poids, de préférence 0,2 à 2 % en poids, de manière particulièrement préférée 0,4 à 1 % en poids, respectivement rapporté au poids de la composition (KR), d'au moins un sel choisi parmi les sels de potassium, de calcium, de strontium, de baryum et d'aluminium avec une solubilité dans l'eau à 20 °C d'au moins 500 mg/l.

3. Procédé selon l'une des revendications 1 ou 2, **caractérisé en ce que** le au moins un sel d'un ion métallique polyvalent choisi parmi les sels de calcium, de strontium, de baryum et d'aluminium avec une solubilité dans l'eau à 20 °C d'au moins 500 mg/l, ainsi que, dans le cas où le polysaccharide formant un gel avec des ions calcium comprend du kappa-carraghénane ou l'un de ses sels, le au moins un sel d'un ion métallique polyvalent choisi parmi les sels de potassium, de calcium, de strontium, de baryum et d'aluminium avec une solubilité dans l'eau à 20 °C d'au moins 500 mg/l, sont introduits dans le procédé par le fait
A. qu'ils sont ajoutés à la composition de mise en forme (KR) déjà lors de la préparation, ou
B. qu'ils se présentent sous forme de poudre et sont ajoutés à la composition de mise en forme (KR) *in situ,* ou
C. qu'ils se présentent sous la forme d'une composition gélifiante à base d'eau (C) et sont ajoutés à la composition de mise en forme (KR) *in situ,* ou
D. qu'ils se présentent sous la forme d'une composition gélifiante à base d'eau (C) et, après l'application de la composition de mise en forme (KR) sur les fibres kératiniques, qu'ils sont également appliqués, notamment pulvérisés, sur les fibres kératiniques.

4. Procédé selon l'une des revendications 1 à 3, **caractérisé en ce que** le au moins un sel d'un ion métallique polyvalent choisi parmi les sels de calcium, de strontium, de baryum et d'aluminium avec une solubilité dans l'eau à 20 °C d'au moins 500 mg/l, ainsi que, dans le cas où le polysaccharide formant un gel avec des ions calcium comprend du kappa-carraghénane ou l'un de ses sels, le au moins un sel d'un ion métallique polyvalent choisi parmi les sels de potassium, de calcium, de strontium, de baryum et d'aluminium avec une solubilité dans l'eau à 20 °C d'au moins 500 mg/l se présentent sous la forme d'une composition gélifiante (C) contenant
d)i. 0,1 à 3 % en poids, de préférence 0,2 à 2 % en poids, de manière particulièrement préférée 0,4 à 1 % en poids, rapporté au poids de la composition (C), d'au moins un sel d'un ion métallique polyvalent choisi parmi les sels de calcium, de strontium, de baryum et d'aluminium avec une solubilité dans l'eau à 20 °C d'au moins 500 mg/l, ainsi que, dans le cas où le polysaccharide formant un gel avec des ions calcium comprend du kappa-carraghénane ou l'un de ses sels, la composition gélifiante (C) contient 0,1 à 3 % en poids, de préférence 0,2 à 2 % en poids, de manière particulièrement préférée 0,4 à 1 % en poids, rapporté au poids de la composition (C), d'au moins un sel choisi parmi les sels de potassium, de calcium, de strontium, de baryum et d'aluminium avec une solubilité dans l'eau à 20 °C d'au moins 500 mg/l,
d)ii. 50 à 99 % en poids, rapporté au poids de la composition (C), d'eau,
d)iii. éventuellement au moins un polymère cationique, qui est de préférence choisi parmi les éthers de guar cationisés ;
d)iv. la composition gélifiante (C) présentant un pH compris entre 4 et 12, de préférence entre 4 et 9, de manière particulièrement préférée entre 6,5 et 8, mesuré à 20 C ; éventuellement la composition gélifiante (C) étant préparée *in situ* ou 0,01 à 24 heures avant la mise en œuvre du procédé de mise en forme selon l'invention en mélangeant une composition gélifiante solide, de préférence en poudre (C'), contenant le au moins un sel mentionné à l'étape d) sous forme pulvérulente et éventuellement au moins un polymère cationique, de préférence choisi parmi les éthers de guar cationisés, sous forme pulvérulente, avec de l'eau d)iii,
**caractérisé en ce que** la composition gélifiante (C), dans une étape de procédé,
- est appliquée, de préférence pulvérisée, après l'étape d'application ii et avant l'application, de préférence la pulvérisation, de la composition de polysaccharide (D) sur les fibres kératiniques soumises à la composition de mise en forme (KR), ou
- est ajoutée à la composition de mise en forme (KR) avant l'étape d'application ii, ou
- est appliquée, de préférence pulvérisée, après l'application, de préférence la pulvérisation, de la composition de polysaccharide (D) sur les fibres kératiniques soumises à la composition de mise en forme (KR).

5. Procédé selon la revendication 3 ou 4, **caractérisé en ce que** le rapport pondéral de la composition gélifiante (C) à la composition de mise en forme (KR) est compris entre 1:20 et 1:2, de préférence entre 1:10 et 1:5.

6. Procédé selon l'une quelconque des revendications 1 à 5, **caractérisé en ce que** le rapport pondéral de la composition de polysaccharide (D) à la composition de mise en forme (KR) est compris entre 1:20 et 1:2, de préférence entre 1:10 et 1:5.

7. Procédé selon l'une quelconque des revendications 3 à 6, **caractérisé en ce que** le rapport pondéral de la composition de polysaccharide (D) à la composition gélifiante (C) est compris entre 0,2:1 et 2:1, de préférence entre 0,5:1 et 1,5:1 et, de manière particulièrement préférée, est de 1:1.

8. Procédé selon l'une quelconque des revendications 1 à 7, **caractérisé en ce que** le au moins un polymère contenant des monomères d'acide (méth)acrylique, d'ester d'acide (méth)acrylique, de (méth)acrylamide et/ou d'acide 2-acrylamido-2-méthylpropanesulfonique est choisi parmi des homopolymères réticulés d'acide acrylique, des homopolymères réticulés d'acide 2-acrylamido-2-méthylpropanesulfonique, des copolymères réticulés constitués de motifs d'acide acrylique et d'acide 2-acrylamido-2-méthylpropanesulfonique, des copolymères réticulés constitués de motifs d'acide 2-méthyl-2[(1-oxo-2-propényl)amino]-1-propanesulfonique et de motifs acrylate d'hydroxyéthyle, des copolymères réticulés constitués de motifs d'acrylamide et de motifs d'acide 2-acrylamido-2-méthylpropanesulfonique, des copolymères réticulés constitués de motifs d'acide méthacrylique et de motifs d'acrylate d'alkyle en C1-C4, des copolymères réticulés constitués de motifs d'acide acrylique et de motifs méthacrylate d'alkyle en C1-C4, ainsi que leurs mélanges.
